# DEMANDE DE BREVET EUROPEEN

(11) **EP 1 438 968 A1**
(43) Date de publication de la demande: **21.07.2004**
(21) Numéro de dépôt: 03293058.8
(22) Date de dépôt: 08.12.2003
(51) Int. Cl.: A61K 38/50, A61K 7/48

(54) **Compositions pour application topique comprenant au moins un polypeptide de la famille des hydrolases à activité amidasique et/ou un produit capable de moduler son activité**

(30) Priorité: 16.01.2003 FR 0300454
(71) Demandeur: L'OREAL, 75008 Paris (FR)
(72) Inventeur: Delattre, Caroline, 75012 Paris (FR); Bernard, Dominique, 75015 Paris (FR); Mehul, Bruno, 06650 Opio (FR)
(74) Mandataire: Allab, Myriam

(57) **Abrégé**

La présente invention a trait à une composition comprenant, dans un milieu physiologiquement acceptable adapté à une application topique sur la peau, au moins un composé choisi parmi (i) un polypeptide de la famille des hydrolases à activité amidasique ou un précurseur de celui-ci et (ii) un produit capable de moduler l'activité dudit polypeptide.

Elle porte également sur l'utilisation de ces compositions, notamment pour le traitement des peaux sèches et/ou des signes du vieillissement cutané.

## Description

La présente invention a trait à une composition comprenant, dans un milieu physiologiquement acceptable adapté à une application topique sur la peau, au moins un composé choisi parmi (i) un polypeptide de la famille des hydrolases à activité amidasique ou un précurseur de celui-ci et (ii) un produit capable de moduler l'activité dudit polypeptide ; et leurs utilisations notamment pour moduler le phénomène de desquamation.

La desquamation est un phénomène naturel lié au fait que l'épiderme, qui constitue la couche supérieure de la peau, est en constante régénération.
L'épiderme est constitué de plusieurs assises de cellules, dont la plus profonde est l'assise basale constituée de cellules indifférenciées. Au cours du temps, ces cellules vont différencier et migrer vers la surface de l'épiderme en constituant les différentes assises de celui-ci, jusqu'à former à la surface de l'épiderme les cornéocytes qui sont des cellules mortes qui s'éliminent par desquamation.
Cette perte en surface est compensée par la migration de cellules de l'assise basale vers la surface de l'épiderme : il s'agit du renouvellement perpétuel de la peau. Une élimination forcée de la couche cornée accélère donc le renouvellement et permet de lutter contre le vieillissement.
Dans le même temps, ces cellules poursuivent leur différenciation dont le dernier stade est le cornéocyte. Il s'agit en fait de cellules mortes qui constituent la dernière couche de l'épiderme, c'est-à-dire la couche la plus externe encore appelée *stratum corneum.*

Le vieillissement cutané résulte de facteurs intrinsèques ou extrinsèques se traduisant par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté.
Certains de ces signes du vieillissement sont plus particulièrement liés au vieillissement intrinsèque ou physiologique, c'est-à-dire au vieillissement « normal » lié à l'âge ou chronobiologique, alors que d'autres sont plus spécifiques du vieillissement extrinsèque, c'est-à-dire du vieillissement provoqué d'une manière générale par l'environnement ; il s'agit plus particulièrement du photovieillissement dû à l'exposition au soleil, à la lumière ou à tout autre rayonnement.

On connaît dans l'art antérieur divers agents aux propriétés desquamantes destinés à lutter contre le vieillissement cutané.
Ainsi, le brevet US-A-4 603 146 décrit l'emploi d'acide rétinoïque et de ses dérivés dans des compositions cosmétiques, en vue de lutter contre le vieillissement cutané.
Par ailleurs, de nombreuses compositions cosmétiques du commerce enseignent l'emploi des α-hydroxyacides comme l'acide lactique, l'acide glycolique ou encore l'acide citrique pour traiter le vieillissement cutané.
On connaît enfin les β-hydroxyacides et plus spécialement l'acide salicylique ainsi que ses dérivés pour leur propriétés desquamantes (WO-A-93/10756 et US-A-4 767 750).
Tous ces composés ont une action contre le vieillissement de la peau en favorisant la desquamation, c'est-à-dire l'élimination des cellules 'mortes' situées à la surface de la couche cornée de l'épiderme. Cette propriété 'desquamante' est aussi appelée, souvent à tort, propriété kératolytique.

Mais les composés de l'art antérieur présentent également des effets secondaires, qui consistent en des picotements, des tiraillements, des échauffements et des rougeurs désagréables pour l'utilisateur.

Il reste donc un besoin de disposer de nouveaux composés efficaces utilisables dans une composition adaptée à une application topique sur la peau, pour faciliter notamment la desquamation de la peau.

Or la demanderesse a trouvé de manière inattendue que l'Aspartylglucosaminidase AGA (EC 3.5.1.26) appartenant à la famille des hydrolases à activité amidasique (EC 3.5.1.X), est présente dans l'épiderme au niveau du *stratum corneum* et a une activité prodesquamante.
Pourtant l'étude de certaines mutations de l'AGA responsables d'une maladie génétique grave appelée l'Aspartylglucosaminuria (AGU), avait montré jusqu'ici une expression de l'AGA principalement au niveau des hépatocytes, des cellules pyramidales du cortex cérébral, des cellules du tubule proximal du rein et dans une moindre mesure, au niveau du tissu conjonctif (Enomaa N.E. et al, (1993), *The Journal of Histochemistry and Cytochemistry,* Vol 41, N°7, pp 981-989 ; Arvio et al, (1999), J Med Genet, 36, 398-404). Aucune mention n'était faite d'une quelconque expression ni activité de l'AGA, *a fortiori* d'une activité prodesquamante, au niveau de l'épiderme, en particulier au niveau du *stratum corneum.*

L'invention concerne donc une composition comprenant, dans un milieu physiologiquement acceptable adapté à une application topique sur la peau, au moins un composé choisi parmi (i) un polypeptide de la famille des hydrolases à activité amidasique ou un précurseur de celui-ci et (ii) un produit capable de moduler l'activité dudit polypeptide.

Elle concerne également l'utilisation desdits composés ou de la composition pour moduler, en particulier favoriser la desquamation et/ou moduler le renouvellement cellulaire de l'épiderme et/ou moduler l'hydratation de la peau et/ou moduler la prolifération et/ou la différenciation cellulaire au niveau de la peau et/ou faciliter la pénétration cutanée d'actifs de compositions cosmétiques et/ou dermatologiques et/ou lutter contre l'adhésion bactérienne.

Selon un premier mode de réalisation, la composition contient à titre de principe actif au moins (i) un polypeptide de la famille des hydrolases à activité amidasique ou un précurseur de celui-ci.

Les hydrolases à activité amidasique (EC 3.5.1.X) agissent sur des liaisons carbone/azote autres que des liaisons peptidiques. En particulier, l'Aspartylglucosaminidase AGA (EC 3.5.1.26) est une enzyme lysosomiale qui hydrolyse les liens N-acetylglucosamine-asparagine des glycopeptides et glycoprotéines humaines (McGovern MM et al, (1983), *The journal of biological chemistry,* 258, 17, 10743-10747).

A titre d'exemples de composés de la famille des hydrolases à activité enzymatiques, on peut citer les composés EC 3.5.1.1 à EC 3.5.1.89 de la nomenclature classique, parmi lesquels l'asparaginase, la glutaminase, l'amidase, l'urease, l'aminoacylase, l'aspartoacylase, la ceramidase, la peptidyl-glutaminase, la formamidase, la pentanamidase, et l'aspartylglucosaminidase AGA.

On utilisera de préférence dans le cadre de l'invention un polypeptide à activité aspartylgucosaminidase ou un précurseur de celui-ci.

Par 'précurseur d'un polypeptide', on entend une forme non active ou 'pro-' de l'enzyme qui, après transformation, conduit à la forme active de l'enzyme. Cette transformation peut consister en une séparation du peptide signal et une autoprotéolyse intramoléculaire.

Dans le cadre de l'invention, ledit polypeptide à activité aspartylglucosaminidase peut être choisi parmi :
a) un polypeptide d'origine humaine ou un homologue dudit polypeptide ayant une activité aspartylglucosaminidase ;
b) un analogue enzymatique ou biomimétique dudit polypeptide selon a), ayant une activité aspartylglucosaminidase;
c) un fragment dudit polypeptide selon a), ledit fragment ayant une activité aspartylglucosaminidase ;
d) un polypeptide ou analogue enzymatique ou fragment de polypeptide selon a), b) ou c) sous une forme active de type hétérodimère ou hétérotétramère ;
e) un polypeptide ou analogue enzymatique ou fragment de polypeptide selon a), b) ou c) ayant subi une ou plusieurs modifications.

Le polypeptide d'origine humaine ayant une activité aspartylglucosaminidase comprend par exemple la séquence peptidique décrite sous le n° d'accession SwissProt P20933 (Fisher et al., 1990, FEBS Letter, 269(2), 440-444) ou ses homologues.

D'une façon générale, on entend par 'homologue' d'un polypeptide ou d'une séquence peptidique, tout polypeptide ou toute séquence peptidique identique à au moins 50%, de préférence à au moins 80% et encore plus préférentiellement à au moins 95 % d'un polypeptide ou d'une séquence peptidique définie, chez la même espèce ou chez une espèce différente ; dans ce dernier cas, on le désigne également par 'polypeptide orthologue'.

A titre de polypeptides à activité aspartylgucosaminidase utilisables selon l'invention, on peut citer par exemple les polypeptides de séquences AGA connues chez les Eucaryotes, en particulier les séquences AGA de mammifères (souris, homme), de levure et de plantes et leurs orthologues (polypeptide homologues, chez une espèce différente), en particulier :

| Espèce | N°d'accession SwissProt |
|---|---|
| *- Caenorhabditis elegans* (nématode) | (Q21697) |
| *- Flavobacterium meningosepticum* (bactérie) | (Q47898) |
| *- Homo sapiens* (humain) | (P20933) |
| *- Mus musculus* (souris) | (Q64191) |
| *- Sus scrofa* (porc) | (P30918) |
| *- Rattus norvegicus* (rat) | (P30919) |
| *- Spodoptera frugiperda* (insecte) | (002467) |

Par 'pourcentage d'identité' entre deux séquences peptidiques ou séquences d'acides aminés, on entend désigner un pourcentage de résidus d'acides aminés identiques entre les deux séquences à comparer, obtenu après le meilleur alignement, c'est-à-dire l'alignement optimal réalisé par exemple au moyen de l'algorithme d'homologie locale de Smith et Waterman (1981, Ad. App. Math. 2 :482), au moyen de l'algorithme d'homologie locale de Neddleman et Wunsch (1970, J.Mol. Biol. 48 : 443), au moyen de la méthode de recherche de similarité de Pearson et Lipman (1988, Proc. Natl. Acad. Sci. USA 85 :2444), au moyen de logiciels informatiques utilisant ces algorithmes (GAP, BESTFIT, BLAST P, BLAST N disponible sur le site NCBI, FASTA et TFASTA dans le Wisconsin Genetics Software Package, Genetics Computer Group, 575 Science Dr, Madison, WI).

Par 'analogues enzymatiques ou biomimétiques' ayant une activité aspartylglucosaminidase, on entend toute molécule capable d'hydrolyser les liaisons carbone-azote autres que les liaisons peptidiques, en particulier des liaisons N-acetylglucosamine-asparagine des glycopeptides et glycoprotéines humaines. On peut citer comme exemples d'analogues enzymatiques ou 'synzymes' :
- des enzymes artificiels qui ont la capacité de catalyser les réactions en se liant à des états de transition du substrat; à titre 'd'hydrolases-like' par exemple, on peut citer les cyclodextrines, les cyclophanes, les porphyrines cycliques ;
- des anticorps catalytiques ou 'abzymes', qui se lient sélectivement à un analogue de l'état de transition du substrat (Schultz PG et al, 1986, Science, 234, 1570-1573), et que l'on peut désormais obtenir par immunisation *in vitro* en utilisant l'analogue stable de l'état de transition comme antigène ;
- des enzymes ARN ou 'ribozymes' qui ont un pouvoir catalytique comme décrit dans Bartel DP et coll, 1993, Science, 251 :1411-1418.

Par 'fragment de polypeptide', on entend tout fragment caractérisé en ce que sa taille permet de reconstituer le site actif de l'enzyme nécessaire à l'activité et à la spécificité de l'aspartylglucosaminidase. L'acide aminé Thréonine 206 est décrit comme nécessaire pour l'activité de l'aspartylglucosaminidase ainsi que l'association de ses deux chaînes alpha et bêta en hétérodimère. On choisira donc des polypeptides de taille comprise entre 1 et 50kD qui préservent cet acide aminé 206. Ce fragment de polypeptide peut être obtenu par protéolyse ou de manière synthétique selon les méthodes connues.

Le polypeptide ou analogue enzymatique ou fragment de polypeptide à activité aspartylglucosaminidase selon l'invention peut être sous la forme d'un précurseur ou sous la forme active d'un hétérodimère ou hétérotétramère.

Le précurseur de l'aspartylglucosaminidase correspond à la forme non active de l'enzyme qui, après séparation du peptide signal et autoprotéolyse intramoléculaire, conduit à deux sous-unités de 24kDa (Alpha) et 18 kDa (Bêta) dont l'association sous forme d'hétérodimère ou hétérotétramère conduit à la forme active de l'enzyme (Saarela J., 1998, *The Journal of Biochemistry,* Vol. 273, N°39, 25320-25328).

On peut également utiliser dans le cadre de l'invention un polypeptide ou analogue enzymatique ou fragment de polypeptide à activité aspartylglucosaminidase selon l'invention ayant subi une ou plusieurs modifications. Par 'modification', on entend toute substitution, délétion et/ou insertion d'un acide aminé ou d'un nombre réduit d'acides aminés, notamment par substitution d'acides aminés naturels par des acides aminés non naturels ou pseudoacides aminés à des positions telles que les modifications ne portent pas significativement atteinte à l'activité biologique de l'AGA. La modification peut aussi correspondre à des substitutions conservatives, c'est-à-dire des substitutions d'acides aminés de même classe, telles que des substitutions d'acides aminés aux chaînes latérales non chargées (asparagine, glutamine, sérine, thréonine, tyrosine), d'acides aminés aux chaînes latérales basiques (lysine, arginine et histidine), d'acides aminés aux chaînes latérales acides (acide aspartique et acide glutamique) ; d'acides aminés aux chaînes latérales apolaires (glycine, alanine, valine, leucine, isoleucine, proline, phénylalanine, méthionine, tryptophane et cystéine).

De préférence, le polypeptide d'origine humaine ayant une activité aspartylglucosaminidase est susceptible d'être isolé et purifié à partir du *stratum corneum* de l'épiderme humain ; il présente une masse moléculaire apparente comprise entre 10 et 50kD, particulièrement entre 15 et 48kD et notamment entre 15 et 24kD. Il peut être obtenu selon les méthodes connues d'extraction et de purification, et en particulier selon le protocole décrit à l'exemple 1 ci-après, comprenant des étapes successives d'extraction en présence d'EDTA, de filtration, de chromatographie cationique et de dosage d'activité.

Le polypeptide appartenant à la famille des hydrolases à activité amidasique et en particulier l'AGA peuvent être d'origine naturelle ou synthétique, en particulier recombinante.
Par 'origine naturelle', on entend un polypeptide à l'état pur ou en solution à différentes concentrations, obtenu par différents procédés d'extraction à partir d'un tissu (peau, foie...) d'origine naturelle, en particulier le *stratum corneum* de l'épiderme humain.
Par 'origine synthétique', on entend un polypeptide à l'état pur ou en solution à différentes concentrations, obtenu chimiquement ou par production dans un organisme après introduction dans cet organisme des éléments nécessaires à cette production.

Dans une composition selon l'invention, le polypeptide de la famille des hydrolases à activité amidasique est généralement en une quantité comprise entre 10⁻⁶ à 5% en poids total de la composition, préférentiellement entre 10⁻⁴ à 1% en poids total de la composition et plus préférentiellement entre 0,001 et 0,1% en poids total de la composition.

Selon une première variante, le polypeptide AGA est maintenu sous la forme d'un précurseur non actif dans la composition jusqu'à son application sur la peau, où les conditions de pH et de force ionique sont favorables à la réaction d'autoprotéolyse nécessaire à l'activation de l'AGA. On sait que l'activation de l'AGA est favorisée et/ou déclenchée à un pH compris entre 5 et 8.

Selon une seconde variante, le polypeptide AGA est sous sa forme active dans la composition, ladite composition contenant déjà les éléments nécessaires à l'autoprotéolyse de la forme précurseur (conditions physiologiques de pH et force ionique favorables). Lesdits éléments favorisant l'activité de l'AGA peuvent se trouver dans le même compartiment que le polypeptide AGA ou dans un compartiment séparé, l'activation de l'AGA étant différée jusqu'à l'application de la dite composition, comme décrit en détail ci-après (mode préféré).

Selon un deuxième mode de réalisation, la composition comprend, à titre de principe actif, au moins (ii) un produit capable de moduler l'activité du polypeptide de la famille des hydrolases à activité amidasique, en particulier de l'AGA.

Par 'moduler l'activité du polypeptide', on entend moduler l'expression dudit polypeptide et/ou moduler l'activité enzymatique dudit polypeptide. Par produit capable de moduler l'activité du polypeptide de la famille des hydrolases à activité amidasique, il faut donc entendre tout élément capable d'augmenter ou de diminuer la quantité dudit polypeptide, soit en stimulant soit en réprimant la synthèse dudit polypeptide, que ce soit par un mécanisme transcriptionnel, post transcriptionnel, translationnel ou post translationnel, soit en stimulant soit en réprimant le catabolisme dudit polypeptide, soit en stimulant soit en réprimant l'activité intrinsèque dudit polypeptide.

Ces produits peuvent être sélectionnés selon un procédé de criblage basé sur la mesure d'une variation de l'expression et/ou de l'activité dudit polypeptide.

A titre d'exemples de procédés bien connus de l'homme du métier, on peut citer :
- des tests de type RT-PCR quantitative tels que décrits dans White B.A., (1997), *Methods on Molecular Biology,* Humana Press, Vol 67 ;
- des tests enzymatiques tels que celui décrit dans Mononen I.T et al, (1993), *Analytical biochemistry,* 208, 372-374 ;
- des test ELISA avec des anticorps spécifiques tels que décrits dans Kemeny D.M., (1991), *A Practical Guide to ELISA,* Pergamon Press.

Ce produit capable de moduler l'activité dudit polypeptide peut être un activateur ou un inhibiteur.

Par 'activateur', on entend soit un produit, soit un ensemble de produits capables de stimuler l'activité dudit polypeptide, par exemple d'augmenter la vitesse de la réaction enzymatique, mesurée par l'augmentation de la quantité de substrats digérés par unité de temps lors de la mise en contact du polypeptide AGA avec l'activateur.

En particulier ces produits pourront agir en modifiant l'environnement de l'enzyme, en particulier les conditions de pH et de force ionique de façon à les rendre favorables à l'activation de ladite enzyme.

Cette mise en contact peut avoir lieu au moment de l'application de la composition contenant l'activateur sur l'épiderme de la peau qui contient, en particulier au niveau du *stratum corneum,* des polypeptides AGA endogènes.

A titre d'exemple d'activateur, on peut utiliser le Sodium Dodécyl Sulfate (SDS) à des concentrations non irritantes de 1 à 5% pour les produits rincés ou préférentiellement le Sodium Lauryl Ether Sulfate à des concentrations non irritantes comprises entre 10 et 25% pour les produits rincés ou entre 0.001 et 0.1% pour les produits de soin.

Ces activateurs représentent généralement entre 0,01% et 50% du poids total de la composition, de manière préférée entre 0,1 et 1% du poids total de la composition. Les plus fortes concentrations sont réservées pour des applications de type 'peeling'. Pour les autres applications cosmétiques, on utilisera préférentiellement des proportions inférieures à 10%, en particulier inférieures à 1%.

Par 'inhibiteur', on entend un produit capable d'inhiber l'activité dudit polypeptide, c'est-à-dire de diminuer la vitesse de la réaction enzymatique, mesurée par exemple par la diminution de la quantité de substrats digérés par unité de temps lorsque ledit produit est mis en contact avec le polypeptide. En particulier, ces produits pourront agir au niveau du site actif de l'enzyme ou par compétition vis-à-vis de la fixation au substrat.

Cette mise en contact peut avoir lieu au moment de l'application de la composition contenant l'inhibiteur sur l'épiderme de la peau qui contient, en particulier au niveau du *stratum corneum,* des polypeptides AGA endogènes. Comme exemples d'inhibiteurs, on peut citer la L-asparagine qui module l'AGA de façon réversible et son analogue le 5-diaxo-4-oxo-L-norvaline, qui module l'AGA de façon irréversible (Noronski et al., *FEBS Letters,* 412, 149-152, 1997). On peut également citer la N-acétylcystéine, l'asparagine, l'aspartylalanine, l'aspartylcyclohexylamine et l'acide p-chloromercuribenzoïque.

Ces inhibiteurs représentent généralement entre 10⁻⁶ et 5% du poids total de la composition, de manière préférée entre 0,1 et 1% du poids total de la composition.

Ces inhibiteurs sont particulièrement intéressants puisqu'ils permettent d'augmenter l'effet barrière de la peau, en favorisant le développement en épaisseur du *stratum corneum* et contribuer ainsi à :
- lutter contre effets néfastes des rayonnements UV impliqués dans le processus de photovieillissement ;
- et/ou lutter contre l'amincissement de la peau qui est un des signes du vieillissement chronobiologique.

L'utilisation des inhibiteurs est particulièrement préconisée dans le traitement des peaux sensibles et/ou âgées et dans le traitement des dermatites atopiques liées à des troubles de la fonction barrière.

Selon un mode préféré, la composition selon l'invention comprend, dans un milieu physiologiquement acceptable adapté à une application topique sur la peau, au moins (i) un polypeptide de la famille des hydrolases à activité amidasique ou un précurseur de celui-ci et (ii) un activateur dudit polypeptide. Une telle association permet de diminuer la concentration active desdits actifs (produit activateur) du fait des effets additifs. On peut ainsi obtenir une composition moins irritante et moins toxique, ainsi qu'une composition plus efficace que celles de l'art antérieur n'utilisant que ces actifs (produit activateur). Cette association est particulièrement intéressante pour des activateurs comme le SDS par exemple, qui peut présenter, à forte concentration, un effet irritant sur la peau.

Les composés (i) et (ii) peuvent être administrés ensemble dans une même composition, ou encore séparément dans des compositions séparées, de manière simultanée ou décalée dans le temps.
Avantageusement, le polypeptide de la famille des hydrolases à activité amidasique et l'activateur dudit polypeptide sont conditionnés de sorte à ne pas être en contact l'un avec l'autre comme décrit par la Demanderesse dans FR-2 780 645, par exemple dans deux compositions différentes, qui peuvent être soit mélangées au moment de l'application soit appliquées de façon successive ou décalée dans le temps.
Par exemple, le polypeptide de la famille des hydrolases à activité amidasique et l'activateur dudit polypeptide sont conditionnés dans des compartiments séparés ou des phases séparées. De tels dispositifs de conditionnement en deux compartiments sont par exemple décrits dans les documents FR-A-2045559, FR-A-2105332, FR-A-2258319, FR-A-2293375, FR-A-2586913 ou FR-A-2643615.
On peut aussi réaliser l'une des compositions sous forme encapsulée et/ou sous forme de microcapsules ou de microgranulés immergés dans l'autre composition, les microcapsules ou les microgranulés étant écrasés au moment de l'application par frottement sur la peau, ce qui permet ainsi le mélange dudit polypeptide de la famille des hydrolases à activité amidasique avec l'activateur dudit polypeptide.

Selon une variante, le polypeptide AGA est sous la forme d'un précurseur non-actif, intégré dans des microcapsules ou des microgranulés immergés dans l'autre composition comprenant les éléments nécessaires à l'activation du précurseur AGA ; cette activation est différée jusqu'à l'application de la composition sur la peau.

L'invention concerne également une composition comprenant au moins un composé choisi parmi (i) un polypeptide de la famille des hydrolases à activité amidasique ou un précurseur de celui-ci et (ii) un activateur dudit polypeptide, caractérisée en ce qu'elle comprend en outre au moins un autre agent desquamant.

Par 'agent desquamant', on entend tout composé capable d'agir :
- soit sur la desquamation en favorisant l'exfoliation ;
- soit sur les enzymes impliquées dans la desquamation ou la dégradation des cornéodesmosomes.

On peut citer par exemple : les β-hydroxyacides, en particulier l'acide salicylique et ses dérivés tels que l'acide n-octanoyl 5-salicylique ; les α-hydroxyacides, tels que les acides glycolique, citrique, lactique, tartrique, malique ou mandélique ; les a- ou β-céto-acides ; l'urée ; l'acide gentisique ; les oligofucoses ; l'acide cinnamique ; l'extrait de Saphora japonica ; le resvératrol et ses dérivés ; les glycosidases, la stratum corneum chymotryptic enzyme (SCCE) voire d'autres protéases à sérine ou cystéine (trypsin, chymotrypsin-like ou encore protéase à cystéine comme décrit par la Demanderesse dans FR-2 767 833) ; les agents chélatant des sels minéraux: l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M®) ; les chélateurs de calcium ; le miel ; les dérivés de sucre tels que l'O-octanoyl-6-D-maltose et la N-acétyl glucosamine ; les agents réducteurs , le glutathion, la cystéine ; ou encore certains carbohydrates tels que ceux définis dans la demande de brevet WO-A-97 12597) ; l'acide jasmonique et ses dérivés ; des rétinoïdes comme l'acide rétinoïque (all-trans ou 13-cis) et ses dérivés, le rétinol (vitamine A) et ses esters tels que le palmitate de rétinol, l'acétate de rétinol et le propionate de rétinol ainsi que leurs sels, ou encore le rétinal.
A titre d'exemple, l'autre agent desquamant peut être introduit dans les compositions utilisées selon l'invention en une quantité représentant de 0,01 à 50% en poids total de la composition et mieux de 0,1 à 3%.

Une telle association avec un polypeptide de la famille des hydrolases à activité amidasique permet de diminuer la concentration active des autres produits à activité desquamante du fait des effets additifs. On peut ainsi obtenir une composition moins irritante et moins toxique, ainsi qu'une composition plus efficace que celles de l'art antérieur n'utilisant que ces actifs.

L'invention porte également sur une composition comprenant au moins un composé choisi parmi (i) un polypeptide de la famille des hydrolases à activité amidasique ou un précurseur de celui-ci et (ii) un produit capable de moduler l'activité du polypeptide, caractérisée en ce qu'elle comprend en outre au moins un composé choisi parmi les agents hydratants ; les agents propigmentants ; les agents stimulant la synthèse de macromolécules dermiques ou épidermiques et/ou empêchant leur dégradation ; les agents stimulant la prolifération ou la différenciation des kératinocytes ; les agents relaxants ; les agents anti-pollution et/ou anti-radicalaire ; les filtres UV ; les agents perméants ; les agents cicatrisants ; et leurs mélanges.

On associera de préférence, dans une composition selon l'invention, les agents hydratants et/ou les agents stimulant la prolifération des kératinocytes et/ou les agents cicatrisants aux composés choisi parmi (i) un polypeptide de la famille des hydrolases à activité amidasique ou un précurseur de celui-ci et (ii) un activateur dudit polypeptide.

Les agents stimulant la synthèse de macromolécules dermiques ou épidermiques et/ou empêchant leur dégradation ; les agents stimulant la différenciation des kératinocytes, les agents anti-pollution et/ou antiradicalaires ; les filtres UV ; les agents perméants ou leurs mélanges seront préférentiellement associés, dans une composition selon l'invention, à un inhibiteur du polypeptide de la famille des hydrolases à activité amidasique.

Par 'agent hydratant', on entend :
- soit un composé agissant sur la fonction barrière, en vue de maintenir l'hydratation du *stratum corneum,* ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du *stratum corneum,* tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sudoripares tels que l'acide caféique ou un composé favorisant la sudation par vasodilatation locale.

Comme 'agent pro-pigmentant', on peut citer l'extrait de pimprenelle (*Sanguisorba officinalis*) commercialisé par la société MARUZEN et les extraits de chrysanthème (*Chrysanthemum morifolium*).

Les 'agents stimulant la prolifération des kératinocytes' comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de *Solanum tuberosum* commercialisés par la société SEDERMA.

Les 'agents stimulant la différenciation des kératinocytes' comprennent par exemple les minéraux tels que le calcium ; l'extrait de lupin commercialisé par la société SILAB sous la dénomination commerciale Photopréventine® ; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl®.

Les 'agents relaxants' comprennent en particulier l'alvérine et ses sels, le manganèse et ses sels ; le magnésium et ses sels ; les sapogénines telles que la diosgénine et les extraits naturels en contenant (tels que les extraits de Wild Yam), ainsi que l'hexapeptide Argireline® commercialisé par la société LIPOTEC et ses sels.

Par 'agent anti-pollution', on entend tout composé capable de piéger l'ozone, les composés aromatiques mono- ou polycycliques tels que le benzopyrène et/ou les métaux lourds tels que le cobalt, le mercure, le cadmium et/ou le nickel.

Par 'agent anti-radicalaire', on entend tout composé capable de piéger les radicaux libres, tels que la vitamine E et ses dérivés ; les bioflavonoïdes ; le coenzyme Q10 ou ubiquinone ; certaines enzymes comme la catalase, la superoxyde dismutase, la lactoperoxydase, la glutathion peroxydase et les quinones réductases ou leurs mimétiques ; le glutathion ; le benzylidène camphre; les benzylcyclanones ; les naphtalénones substituées ; les pidolates ; le phytantriol ; le gamma-oryzanol ; les lignanes ; et la mélatonine.

La composition selon l'invention peut également renfermer des 'filtres UVA et/ou UVB', sous forme de composés organiques ou inorganiques, ces derniers étant éventuellement enrobés pour les rendre hydrophobes.

Les filtres organiques peuvent notamment être choisis parmi : les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-3 (Oxybenzone), ou la Benzophénone-4 (Uvinul MS40 disponible chez B.A.S.F.); les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 disponible chez Merck); les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.); les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane.

Les filtres inorganiques sont de préférence constitués d'oxyde de zinc et/ou de dioxyde de titane, de préférence de taille nanométrique, éventuellement enrobés d'alumine et/ou d'acide stéarique.

On peut avantageusement ajouter des 'agents pro-pénétrants ou perméants' pour renforcer la pénétration des agents actifs. On peut citer par exemple des systèmes binaires (US 4,537,776) ou des solvants comme le DMSO (US 3,551,554) comme véhicules, ou encore des agents perméants actifs qui existent à la fois sous forme de base libre et sous forme de sel d'addition d'acide comme décrit dans EP 0 321 870 B1.

On peut également utiliser des 'agents cicatrisants', c'est-à-dire des agents qui favorisent la cicatrisation, en particulier la cicatrisation cutanée, en favorisant la reépidermisation (reépithélialisation et normalisation de l'épiderme et du derme) et/ou en limitant le phénomène de rétraction de la plaie. On peut notamment citer des inhibiteurs de l'activité de l'acide rétinoïque décrits par la Demanderesse dans FR 2 753 627.

La composition utilisée selon l'invention contient un milieu cosmétiquement ou dermatologiquement acceptable, c'est-à-dire un milieu compatible avec la peau, les ongles, les muqueuses, les tissus et les cheveux. Selon un mode préféré de réalisation de l'invention, la composition a un pH permettant une activité optimale des polypeptides utilisés et de préférence proche de celui de la peau, compris entre 5 et 8.

De façon connue, la composition utilisée selon l'invention peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges et les matières colorantes. Les quantités de ces différents adjuvants sont celles classiquement utilisées dans les domaines considérés, et par exemple de 0,01% à 20% du poids total de la composition. Bien entendu, l'homme de l'art veillera à choisir ce ou ces éventuels additifs et/ou leurs quantités de manière telle que les propriétés avantageuses attachées intrinsèquement à la composition conforme à l'invention, en particulier les activités enzymatiques des polypeptides selon l'invention, ne soient pas, ou substantiellement pas, altérées par la ou les adjonctions envisagées.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer le Polysorbate 60 et le stéarage de sorbitane vendus respectivement sous les dénominations commerciales Tween 60 et Span 60 par la Société ICI. On peut y ajouter des co-émulsionnants tels que le PPG-3 myristyl éther vendu sous la dénomination commerciale Emcol 249-3K par la société Witco.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sols métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

La composition cosmétique selon l'invention se présente de préférence sous une forme appropriée pour une administration par voie topique et pour contenir des actifs de types enzymes, comme le polypeptide de la famille des hydrolases à activité amidasique selon l'invention, qui présentent une instabilité en milieu aqueux. De préférence donc, le polypeptide est sous une forme stabilisée.

La composition se présente notamment sous forme de solutions hydroalcooliques ou huileuses, de dispersions du type lotion ou sérum, de gels anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de micro-émulsions, ou encore de microcapsules, de micro-particules, ou de dispersions vésiculaires de type ionique et/ou non ionique. Ces compositions sont préparées selon les méthodes usuelles. Une forme préférée et spécialement adaptée pour des compositions comprenant des actifs enzymes est une forme de type E/H/E telle que décrite dans la demande EP 0 779 071.

Les polypeptides de la famille des hydrolases à activité amidasique peuvent également être sous forme immobilisée sur des supports polymériques tel que décrit dans DE-18824072 ou dans des microcapsules. On peut notamment utiliser des sphères de poly-beta-alanine (liaison covalente) ou des liposomes/niosomes (compartiments). Une autre solution consiste à les incorporer dans un milieu liquide anhydre (US-A-5322683). On peut également utiliser des tensioactifs pour la stabilisation desdits polypeptides en milieu aqueux, ou des polyols associés à un agent structurant comme décrit dans FR-2 737 115.

La composition peut se présenter sous la forme d'une lotion, d'une crème, d'un lait, d'un gel, de mousses pour le soin de la peau, des muqueuses et/ou des fibres kératiniques , ou pour le nettoyage de la peau, d'un masque, de microsphères ou nanosphères ou de dispersions vésiculaires constituées de lipides ioniques (liposomes) et/ou de lipides non ioniques. Les compositions peuvent également consister en des préparations solides constituant des savons ou des pains de nettoyage.

Un autre objet de l'invention est un procédé de traitement cosmétique des peaux sèches, destiné à lutter contre les troubles cutanés liés à la desquamation et/ou au renouvellement cellulaire de l'épiderme et/ou à l'hydratation de la peau et/ou à la prolifération et/ou la différenciation cellulaire au niveau de la peau, caractérisé en ce que l'on applique sur la peau, les muqueuses et/ou les fibres kératiniques, une composition comprenant au moins un composé choisi parmi (i) un polypeptide de la famille des hydrolases à activité amidasique ou un précurseur de celui-ci et (ii) un activateur dudit polypeptide.

Ce procédé vise en particulier :
- à favoriser la desquamation, notamment par hydrolyse des glycoprotéines au niveau des cornéodesmosomes ;
- et/ou à favoriser l'hydratation de la peau et le statut osmotique de l'épiderme, notamment par libération de chaînes sucrées de bas poids moléculaire ;
- et/ou à favoriser le renouvellement cellulaire et/ou la prolifération et/ou différenciation cellulaire au niveau de la peau, notamment par la libération de facteurs de croissance de l'épiderme, comme les amphiregulines ou le facteur de croissance épidermal (Epidermal Growth Factor EGF).

L'invention concerne également un procédé de traitement cosmétique des peaux sensibles et/ou des peaux âgées, destiné à augmenter l'effet barrière de la peau, caractérisé en ce que l'on applique sur la peau, les muqueuses et/ou les fibres kératiniques, une composition comprenant au moins un inhibiteur du polypeptide de la famille des hydrolases à activité amidasique.

Ces procédés consistent en l'application des compositions de l'invention, selon la technique d'utilisation habituelle de ces compositions. Par exemple : application de crèmes, de gels, de sérums, de pommades, de lotions, de laits sur la peau, le cuir chevelu, les ongles et/ou les muqueuses.

L'invention concerne encore l'utilisation d'une composition comprenant au moins un composé choisi parmi (i) un polypeptide de la famille des hydrolases à activité amidasique ou un précurseur de celui-ci et (ii) un activateur dudit polypeptide pour la préparation d'une composition pharmaceutique destinée à traiter les troubles de la desquamation, en particulier l'hyperkératose, la xérose, les ichtyoses, le psoriasis ou les kératoses réactionnelles.

L'invention porte également sur l'utilisation d'une composition comprenant au moins un composé choisi parmi (i) un polypeptide de la famille des hydrolases à activité amidasique ou un précurseur de celui-ci et (ii) un activateur dudit polypeptide pour la préparation d'une composition pharmaceutique destinée à favoriser la cicatrisation, du fait que la dégradation des protéoglycanes de la matrice cellulaire induit une recolonisation des espaces cellulaires par les kératinocytes.

Rentre également dans le cadre de l'invention l'utilisation d'une composition comprenant au moins un inhibiteur du polypeptide de la famille des hydrolases à activité amidasique, pour la préparation d'une composition pharmaceutique destinée à traiter les dermatites atopiques.

L'invention concerne également l'utilisation dans une composition cosmétique pour application topique d'au moins un composé choisi parmi (i) un polypeptide de la famille des hydrolases à activité amidasique ou un précurseur de celui-ci et (ii) un activateur dudit polypeptide, lesdits composés ou ladite composition étant destinés à favoriser la desquamation et/ou l'hydratation de la peau et/ou le renouvellement cellulaire au niveau de la peau et/ou la prolifération et/ou différenciation cellulaire au niveau de la peau.

Rentre également dans le cadre de l'invention l'utilisation dans une composition cosmétique pour application topique d'au moins un composé choisi parmi (i) un polypeptide de la famille des hydrolases à activité amidasique ou un précurseur de celui-ci et (ii) un activateur dudit polypeptide, lesdit composés ou ladite composition étant destinés à faciliter la pénétration cutanée d'actifs de compositions cosmétiques et/ou dermatologiques ; la desquamation ayant pour effet de diminuer l'imperméabilité du *stratum corneum* qui fait obstacle normalement au passage de produits à travers la peau ; la couche de *stratum corneum* joue en effet un rôle de barrière, essentiel pour l'organisme, car elle empêche d'une part l'entrée des pathogènes et autres produits toxiques et d'autre part la sortie de fluides physiologiques.

L'invention concerne aussi l'utilisation dans une composition cosmétique pour application topique d'au moins un composé choisi parmi (i) un polypeptide de la famille des hydrolases à activité amidasique ou un précurseur de celui-ci et (ii) un activateur dudit polypeptide, lesdits composés ou ladite composition étant destinés à lutter contre l'adhésion bactérienne, notamment par libération de chaînes sucrées de bas poids moléculaire et donc modification, au niveau des cornéocytes, des points d'adhésion et de colonisation des bactéries.

La partie qui suit présente la mise en évidence de la présence et de l'activité prodesquamante de l'AGA au niveau du *stratum corneum.* Elle présente par ailleurs des exemples de formulation de compositions selon l'invention sans restreindre la portée de l'invention.

La figure 1 est un histogramme présentant le dosage de l'activité AGA avec le substrat Asp-AMC sur peau reconstruite Episkin® , poudre acétonique ou *Stratum corneum* plantaire.

La figure 2 est un histogramme présentant la variation du signal de la cornéodesmosine après incubation de différentes quantités d'AGA semi-purifiée avec une poudre acétonique pendant 8 heures, contrôle tampon d'incubation retranché.

### EXEMPLES

### Exemple 1 : Démonstration de la présence d'une activité AGA dans le stratum corneum SC

### a) Purification et identification de l'AGA à partir du SC

Un extrait de SC est obtenu par 'grattage' de la face antérieure de la jambe avec un tampon d'extraction à pH 4 contenant 50 mM d'acétate de sodium, 5 mM d'EDTA (Ethylènediaminetétra acétate) et 0.1 % de Tween 20. Après une série de filtrations, l'extrait est séparé par chromatographie cationique ; l 'élution est faite par un gradient linéaire en NaCl dans le même tampon d'extraction.

La purification de l'AGA est suivie par un dosage d'activité avec le substrat fluorescent L ―Aspartic acid bêta (7-amido-4-méthylcoumarin) (Asp-AMC) selon la méthode décrite par Mononen I. T. et al, 1993, Analytical biochemistry, 208, 372-374. 10 µl de chaque fraction éluée sont incubés 24 h à 37°C avec 100 µl de substrat Asp-AMC dilué à 1 mM dans du tampon Tris-HCI 0.1 M, pH 8. La fluorescence est quantifiée au lecteur Biolumin (excitation/émission: 340/450 nm).
L'ensemble des fractions présentant une activité de type AGA (fractions 14 à 43) est ensuite poolé et concentré. Un changement de tampon est effectué sur une colonne EconoPac10DG (Bio-Rad) avec du tampon à pH 7 contenant 50 mM de phosphate de sodium, 5 mM d'EDTA, 150 mM de NaCl et 0.1 % de Tween 20. Une séparation chromatographique de type gel filtration (G200) est réalisée et l'activité AGA de chaque fraction est dosée avec le substrat Asp-AMC.

Un extrait d'AGA semi-purifié est obtenu en poolant les fractions à forte activité AGA (fractions 57 à 71).
Il est ensuite concentré et analysé sur gel après une séparation SDS-PAGE (gel à 12%) et une coloration au nitrate d'argent.
4 bandes majoritaires de poids moléculaire respectifs de 48, 24, 16 et 15 kDa ont été observées. Chaque bande a été analysée par spectrométrie de masse de type MALDI-TOF après une digestion trypsique. L'analyse par cartographie peptidique dans les banques de données NCBI utilisant le logiciel PROFOUND® a conduit à l'identification de la chaîne A de l'AGA (bande de 24 kDa avec un taux de recouvrement de 28 % et une probabilité de 35 %) et de la chaîne B de l'AGA (bandes de 16 et de 15 kDa avec un taux de recouvrement respectif de 64% et 38% et une probabilité de 100% et de 11%).

Un séquençage chimique de type Edman a confirmé l'identification de l'AGA pour ces mêmes bandes.
En particulier, le polypeptide isolé du *stratum corneum* a une séquence peptidique contenant au moins l'enchaînement des acides aminés n° 136-150 de la séquence peptidique humaine sous le numéro de référence SwissProt P20933.

### b) présence de l'AGA sous sa forme active

### - Etudes enzymatiques

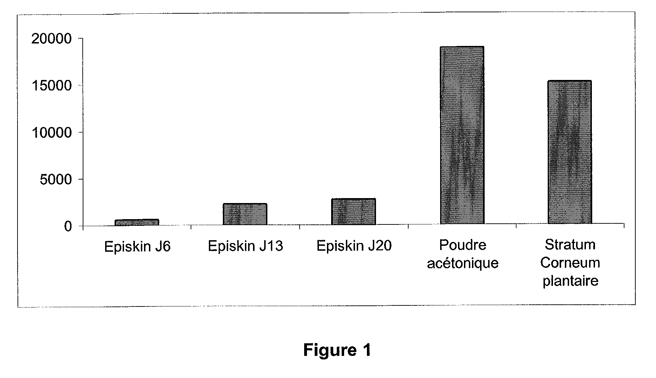

Des études enzymatiques utilisant des substrats et des inhibiteurs spécifiques ont de plus permis de démontrer que l'AGA était présente dans le SC au moins en partie sous sa forme active, hétérodimère ou hétérotétramère. L'activité AGA a été dosée avec le substrat Asp-AMC dans différents extraits de SC. Une activité de type AGA a été mise en évidence dans des extraits protéiques de peau reconstruite Episkin® , de poudre acétonique obtenue selon la technique du 'stripping vernis' décrite par Mehul et al. (2000, The Journal of Biological Chemistry, Vol 275, N°17, 12841-12847), et de *Stratum corneum* plantaire. Ces résultats sont présentés sur la figure 1.
De plus des inhibiteurs spécifiques tels que la L-asparagine utilisée à 1.25mM inhibe l'activité AGA purifiée à partir du SC.

### - Expression (Western)

Deux anticorps de lapin anti-AGA humaine ont été réalisés : ce sont des anticorps polyclonaux dirigés contre un peptide particulier dans la séquence de la protéine respectivement sur la chaîne alpha et bêta de l'AGA.
La présence de l'AGA a été mise en évidence par immunodétection sur différents extraits de SC après une séparation SDS-PAGE suivie d'un transfert sur membrane de PVDF. L'AGA est immunodétectée avec chaque anticorps dilué au 1/1000^{ème} et un anticorps secondaire anti-lapin couplé à la peroxydase est utilisé pour la révélation avec le réactif chemiluminescent ECL.
L'anticorps dirigé contre la chaine alpha de l'AGA détecte une bande de poids moléculaire de 46-50kDa sur les extraits de SC de type Episkin® , poudre acétonique et *Stratum corneum* plantaire. Une bande de poids moléculaire de 25kDa est également détectée pour un extrait de poudre acétonique. L'anticorps dirigé contre la chaine béta de l'AGA détecte un ensemble de bandes vers 46-50kDa également.

### c) Dosage de l'activité sur différents donneurs

L'activité AGA a été dosée directement dans le SC à partir de prélèvements de type adhésifs 'blenderms' au niveau de l'avant-bras, du front et de la jambe de trois donneurs différents avec le susbtrat Asp-AMC cité précédemment. Chaque activité est normalisée par rapport à la quantité de protéine de chaque prélèvement.
Les résultats des trois donneurs montrent que l'AGA est directement dosable et détectable sur des prélèvements de SC et que l'on n'observe pas de différence importante d'activité selon le donneur ou la zone du prélèvement dans nos conditions expérimentales.

### Exemple 2 : Démonstration de l'effet prodesquamant de l'AGA

Une poudre acétonique de SC obtenue par la technique du 'stripping-vernis' telle que décrite par Mehul B. et al. (2000, The Journal of Biological Chemistry, Vol 275, N°17, 12841-12847) est incubée avec un extrait d'AGA semi-purifiée en présence ou non de L-asparagine à 5 mM à raison de 100 µl de solution d'incubation par mg de poudre acétonique. Les incubations sont réalisées dans du tampon Tris-HCl 0.1 M, pH8, à 37°C, pendant 8 heures, sous agitation. Un contrôle t0 est réalisé ainsi qu'un témoin : tampon seul.

Les protéines solubles de chaque échantillon sont ensuite extraites dans des conditions dénaturantes (SDS, DTT et ébullition). Les protéines totales sont dosées selon la méthode de Bradford et les concentrations sont alignées à 0.6mg/ml dans du tampon d'extraction Laemmli.

Une séparation SDS-PAGE est ensuite réalisée suivie d'un transfert sur membrane de PVDF commercialisée par Millipore. La cornéodesmosine est immunodétectée avec l'anticorps monoclonal G3619 décrit dans Serre G. et al. (1991, J. Invest. Dermatol., 97, 1061-1072) dilué au 1/12500^{ème}. Un anticorps secondaire anti-souris couplé à la peroxydase est utilisé ainsi que le réactif chemiluminescent ECL commercialisé par Ammersham Biosciences pour la révélation des blots.
Chaque blot est ensuite coloré avec une solution d'amidoblack à 0.03% (SIGMA) afin de normaliser la quantité de cornéodesmosine par rapport à une quantification des kératines.

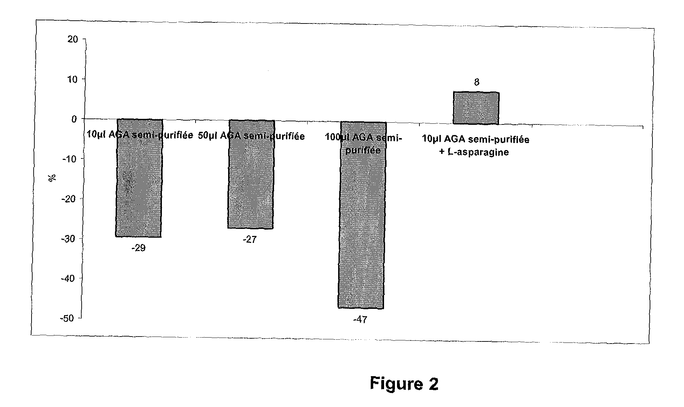

La figure 2 présente la variation du signal de la cornéodesmosine après incubation de différentes quantités d'AGA semi-purifiée avec une poudre acétonique pendant 8 heures.
On observe une diminution de la quantité de cornéodesmosine après incubation avec des quantités croissantes d'AGA.
La présence de L-asparagine à 5 mM inhibe cet effet pour la condition 10µl d'AGA.

Ces résultats permettent donc de démontrer l'effet prodesquamant de l'AGA via l'élimination des cornéocytes.

### Exemple 3: Utilisation dans une composition cosmétique- Exemples de formulation

| **Composition 1 : Lait pour le visage** | |
|---|---|
| Huile de vaseline | 7,0 g |
| AGA | 0,001 g |
| Monostéarate de glycéryle, stéarate de polyéthylène glycol(1 00 OE) | 3,0 g |
| Polymère carboxyvinylique | 0,4 g |
| Alcool stéarylique | 0,7 g |
| Protéines de soja | 3,0 g |
| NaOH | 0,4 g |
| Conservateur | qs |
| Eau | qsp 100 g |

Cette composition se présente sous forme d'un lait pour le visage, ayant de bonnes propriétés cosmétiques, et étant doux et confortable à utiliser.
Le pH de la composition est d'environ 6.

| **Composition 2 : Lotion** | |
|---|---|
| AGA | 0,01 g |
| Palmitate d'éthyl-2 hexyle | 10,0 g |
| Cyclopentadiméthylsiloxane | 20,0 g |
| Butylène glycol | 5,0 g |
| Conservateur | qs |
| Eau | qsp 100 g |

Cette lotion, qui ne contient pas de tensioactif, favorise la desquamation de la peau.

| **Composition 3 : Lait** | |
|---|---|
| Palmitate d'octyle | 35,0 g |
| Glycérine | 2,0 g |
| AGA | 0,1 g |
| Polymère réticulé acrylates C10-C30/alkylacrylates | 0,1 g |
| Triéthanolamine | 0,1 g |
| Acides aminés de blé | 1,0 g |
| Précurseur vit C (molécule bioconvertible) | 0,5g |
| Conservateur | qs |
| Eau | qsp 100 g |

Le lait obtenu, possède de bonnes propriétés cosmétiques.

| **Composition 4 : Gel pour le visage** | |
|---|---|
| Glycérine | 10,0 g |
| Cocoamphodiacétate de disodium | 1,0 g |
| L-asparagine (inhibiteur AGA) | 0,1g |
| Conservateur | qs |
| Eau | qsp 100 g |

Le gel obtenu possède de bonnes propriétés cosmétiques.

| **Composition 5 : Gel nettoyant à l'eau** | |
|---|---|
| Butylène glycol | 7,0 g |
| Sarcosinate de lauroyl sodium | 4,0 g |
| Amidase (famille des hydrolases à activité amidasique) | 0,1 g |
| Triéthanolamine | 0,8 g |
| Carbomer | 0,5 g |
| Conservateur | qs |
| Eau | qsp 100 g |

Le gel obtenu possède de bonnes propriétés cosmétiques.

## Revendications

1. Composition comprenant, dans un milieu physiologiquement acceptable adapté à une application topique sur la peau, au moins un composé choisi parmi (i) un polypeptide de la famille des hydrolases à activité amidasique ou un précurseur de celui-ci et (ii) un produit capable de moduler l'activité dudit polypeptide.

2. Composition selon la revendication 1, **caractérisée en ce que** le polypeptide de la famille des hydrolases à activité amidasique est un polypeptide à activité aspartylglucosaminidase ou un précurseur de celui-ci.

3. Composition selon la revendication 2, **caractérisée en ce que** ledit polypeptide à activité aspartylgucosaminidase est choisi parmi :
a) un polypeptide d'origine humaine ou un homologue dudit polypeptide ayant une activité aspartylglucosaminidase ;
b) un analogue enzymatique ou biomimétique dudit polypeptide selon a), ayant une activité aspartylglucosaminidase;
c) un fragment dudit polypeptide selon a), ledit fragment ayant une activité aspartylglucosaminidase ;
d) un polypeptide ou analogue enzymatique ou fragment de polypeptide selon a), b) ou c) sous une forme active de type hétérodimère ou hétérotétramère ;
e) un polypeptide ou analogue enzymatique ou fragment de polypeptide selon a), b) ou c) ayant subi une ou plusieurs modifications.

4. Composition selon la revendication 3, **caractérisée en ce que** le polypeptide d'origine humaine ayant une activité aspartylglucosaminidase est susceptible d'être isolé et purifié à partir du *stratum corneum* de l'épiderme humain et présente une masse moléculaire apparente comprise entre 10 et 50kD, particulièrement entre 15 et 48kD et notamment entre 15 et 24kD.

5. Composition selon l'une quelconque des revendications 1 à 4, **caractérisée par le fait que** le polypeptide de la famille des hydrolases à activité amidasique est un polypeptide d'origine naturelle.

6. Composition selon l'une quelconque des revendications 1 à 4, **caractérisée par le fait que** le polypeptide de la famille des hydrolases à activité amidasique est un polypeptide recombinant.

7. Composition selon l'une quelconque des revendications 1 à 6, **caractérisée en ce que** le polypeptide de la famille des hydrolases à activité amidasique est en une quantité comprise entre 10⁻⁶ à 5% en poids total de la composition, préférentiellement entre 10⁻⁴ à 1% en poids total de la composition et plus préférentiellement entre 0,001 et 0,1%.

8. Composition selon la revendication 1, **caractérisée en ce que** le produit capable de moduler l'activité dudit polypeptide de la famille des hydrolases à activité amidasique est un activateur capable de stimuler l'activité dudit polypeptide.

9. Composition selon la revendication 8, **caractérisée en que** l'activateur est en une quantité comprise entre 0,01% et 50% du poids total de la composition, de manière préférée entre 0,1 et 1% du poids total de la composition.

10. Composition selon l'une quelconque des revendications 1 à 9, **caractérisée en ce qu'**elle comprend au moins (i) un polypeptide de la famille des hydrolases à activité amidasique ou un précurseur de celui-ci et (ii) un activateur dudit polypeptide.

11. Composition selon la revendication 10, **caractérisée en ce que** le polypeptide de la famille des hydrolases à activité amidasique et l'activateur dudit polypeptide sont conditionnés de sorte à ne pas être en contact l'un avec l'autre.

12. Composition selon l'une quelconque des revendications 8 à 11, **caractérisée en ce qu'**elle comprend en outre au moins un autre agent desquamant, tel que les β-hydroxyacides; les α-hydroxyacides; les a- ou β-céto-acides ; l'urée ; l'acide gentisique ; les oligofucoses ; l'acide cinnamique ; l'extrait de Saphora japonica; le resvératrol et ses dérivés ; les glycosidases, la stratum corneum chymotryptic enzyme (SCCE) ou d'autres protéases à sérine ou à cystéine; les agents chélatants ; les composés aminosulfoniques; les dérivés de sucre ; les agents réducteurs et les rétinoïdes.

13. Composition selon la revendication 1, **caractérisée en ce que** le produit capable de moduler l'activité dudit polypeptide de la famille des hydrolases à activité amidasique est un inhibiteur.

14. Composition selon la revendication 13, **caractérisée en ce que** l'inhibiteur est en une quantité comprise entre 10⁻⁶ et 5% du poids total de la composition, de manière préférée entre 0,1 et 1% du poids total de la composition.

15. Composition selon l'une quelconque des revendications 1 à 14, **caractérisée en ce qu'**elle comprend en outre au moins un composé choisi parmi les agents hydratants ; les agents propigmentants ; les agents stimulant la synthèse de macromolécules dermiques ou épidermiques et/ou empêchant leur dégradation ; les agents stimulant la prolifération ou la différenciation des kératinocytes ; les agents relaxants ; les agents anti-pollution et/ou anti-radicalaire ; les filtres UV ; les agents perméants ; les agents cicatrisants ; et leurs mélanges.

16. Procédé de traitement cosmétique des peaux sèches, destiné à lutter contre les troubles cutanés liés à la desquamation et/ou au renouvellement cellulaire de l'épiderme et/ou à l'hydratation de la peau et/ou à la prolifération et/ou différenciation cellulaire au niveau de la peau, **caractérisé en ce que** l'on applique sur la peau, les muqueuses et/ou les fibres kératiniques, une composition telle que définie dans l'une quelconque des revendications 8 à 12.

17. Procédé de traitement cosmétique des peaux sensibles et/ou des peaux âgées, destiné à augmenter l'effet barrière de la peau, **caractérisé en ce que** l'on applique sur la peau, les muqueuses et/ou les fibres kératiniques, une composition telle que définie dans l'une quelconque des revendications 13 et 14.

18. Utilisation d'une composition comprenant au moins un composé choisi parmi (i) un polypeptide de la famille des hydrolases à activité amidasique ou un précurseur de celui-ci et (ii) un activateur dudit polypeptide, pour la préparation d'une composition pharmaceutique destinée à traiter les troubles de la desquamation, en particulier l'hyperkératose, la xérose, les ichtyoses, le psoriasis ou les kératoses réactionnelles.

19. Utilisation d'une composition comprenant au moins un composé choisi parmi (i) un polypeptide de la famille des hydrolases à activité amidasique ou un précurseur de celui-ci et (ii) un activateur dudit polypeptide, pour la préparation d'une composition pharmaceutique destinée à favoriser la cicatrisation.

20. Utilisation d'une composition comprenant au moins un inhibiteur de polypeptide de la famille des hydrolases à activité amidasique, pour la préparation d'une composition pharmaceutique destinée à traiter les dermatites atopiques.

21. Utilisation dans une composition cosmétique pour application topique d'au moins un composé choisi parmi (i) un polypeptide de la famille des hydrolases à activité amidasique ou un précurseur de celui-ci et (ii) un activateur dudit polypeptide, lesdits composés ou ladite composition étant destinés à favoriser la desquamation et/ou l'hydratation de la peau et/ou le renouvellement cellulaire au niveau de la peau et/ou la prolifération et/ou différenciation cellulaire au niveau de la peau.

22. Utilisation dans une composition cosmétique pour application topique d'au moins un composé choisi parmi (i) un polypeptide de la famille des hydrolases à activité amidasique ou un précurseur de celui-ci et (ii) un activateur dudit polypeptide, lesdits composés ou ladite composition étant destinés à faciliter la pénétration cutanée d'actifs de compositions cosmétiques et/ou dermatologiques.

23. Utilisation dans une composition cosmétique pour application topique d'au moins un composé choisi parmi (i) un polypeptide de la famille des hydrolases à activité amidasique ou un précurseur de celui-ci et (ii) un activateur dudit polypeptide, lesdits composés ou ladite composition étant destinés à lutter contre l'adhésion bactérienne au niveau de la peau.
